# EUROPEAN PATENT SPECIFICATION

(11) **EP 2 884 974 B1**
(45) Date of publication and mention of the grant of the patent: **22.02.2017**
(21) Application number: 14793900.3
(22) Date of filing: 20.08.2014
(51) Int. Cl.: A61K 31/40, A61K 31/593, A61K 31/65, A61K 31/708, A61P 37/00

(54) **USE IN ONE PILL/TABLET/CAPSULE MINOCYCLINE, ACYCLOGUANOSINE, ATORVASTATIN AND VITAMIN D3 IN THE TREATMENT OF RHEUMATOID ARTHRITIS**
MINOCYCLIN, ACYCLOGUANOSIN, ATORVASTATIN UND VITAMIN D3 IN FORM EINER PILLE/TABLETTE/KAPSEL ZUR BEHANDLUNG VON RHEUMATOIDER ARTHRITIS
UTILISATION SOUS FORME DE PILULES/COMPRIMÉS/CAPSULES DE MINOCYCLINE, ACICLOGUANOSINE, ATORVASTATINE ET VITAMINE D DANS LE TRAITEMENT DE L'ARTHRITE RHUMATOÏDE

(30) Priority: 18.08.2014 IT TR20140007
(43) Date of publication of application: 24.06.2015
(73) Proprietor: De Silvestri, Fabrizio, 05100 Terni (IT); Romani, Edoardo, 05029 San Gemini (TR) (IT)
(72) Inventor: De Silvestri, Fabrizio, 05100 Terni (IT); Romani, Edoardo, 05029 San Gemini (TR) (IT)
(86) International application number: PCT/IT2014/000223
(87) International publication number: WO 2015/037023

(56) References cited:
- US-A1- 2007 135 504
- JEONG YEON KIM ET AL: "Atorvastatin inhibits osteoclastogenesis by decreasing the expression of RANKL in the synoviocytes of rheumatoid arthritis", ARTHRITIS RESEARCH AND THERAPY, BIOMED CENTRAL, LONDON, GB, vol. 14, no. 4, 17 August 2012 (2012-08-17), page R187, XP021123320, ISSN: 1478-6354, DOI: 10.1186/AR4018
- HAGA, H. ET AL.: "Severe deficiency of 25-hxdroxyvitamin D3 (25-OH-D3) is associated with high disease activity of rheumatoid arthritis", CLIN RHEUMATOL, vol. 32, 15 January 2013 (2013-01-15), pages 629-633, XP002733160,
- OGRENDIK, M.: "Antibiotics for the treatment of rheumatoid arthritis", INTERNATIONAL JOURNAL OF GENERAL MEDICINE, vol. 7, 27 December 2013 (2013-12-27), pages 43-47, XP002733161,

## Description

TECHNICAL FIELD: new combination drug for the treatment of disability in rheumatoid arthritis and its dosage.

The present invention has per object minocycline, acycloguanosine, atorvastatin and vitamin D3 for use in the preparation of a single composition addressed to treatment of autoimmune type of rheumatoid arthritis in the three stages of development, whether it is active or quiescent phase.

### BACKGROUND ART

The following articles have represented the starting point of this application: "Atorvastatin inhibits osteoclastogenesis by decreasing the expression of RANKL in the synoviocytes of rheumatoid arthritis" (Jeong Yeon Kim et al, 2012), "Severe deficiency of 25-hydroxyvitamin D3 (25-OH-D3) is associated with high disease activity of rheumatoid arthritis" (Haga H. et al, 2012) and "Antibiotics for the treatment of rheumatoid arthritis" (Ogrendik M. , 2013)

Rheumatoid arthritis (RA) is a chronic inflammatory polyarthritis, spondylitis and progressive autoimmune disease of unknown etiology and at the expense of the synovial joints.It differs from osteoarthritis because initially interesting the synovial membrane and cartilage and affects less frequently and at a younger age than osteoarthritis.

Rheumatoid arthritis is a chronic disease that is estimated to affect between 0.3 and 1.0% of the world population. The disease often affects women who are generally four times more affected than men. Pain, fatigue and depression accompany the disease, characterized by progressive damage to articular anatomy resulting in disability, to date clinically assessed with the EDSS (Expanded Disabilty Status Scale), decreased quality and life expectancy. Rheumatoid arthritis is a chronic disease, although spontaneous remission may occur in rare cases after conducting a pregnancy: during pregnancy the hormonal changes may temporarily block the inflammatory state and in rare cases there is a definite remission.

Rheumatoid arthritis affects the joints, making it difficult for the sliding of the bone joints, leading to degenerative medical condition that manifests itself at the level of the cartilage and the synovial membrane that is in contact with the area exposed to inflammation. It's not know the cause for sure, but is evaluated genetic influence (association with MHC-II antigens and in particular HLA-DR1 andHLA-DR4). It is also important positivity for approximately 80% of patients in the rheumatoid factor, demonstrated in the laboratory with the determination of rheumatoid factors is the method to latex that of sheep red blood cells according to the technique of Waaler Rose & changed. Both measure IgM antibodies may be present even if IgA antibodies and IgG. It also reported a second diagnostic marker, the anti-cyclic citrullinated peptides, which seems to have a role in the early diagnosis of the disease. It is also estimated the involvement of Epstein-Barr virus or Mycobacterium tuberculosis. It may therefore also be a microorganism to bring forth the inflammatory reaction in the immune system, which is only for a limited period of time, usually the result of diseases such as mononucleosis that often is confused with disease states such as seasonal influenza viral. It is hypothesized that superantigens may be involved as well as self-antigens (collagen, proteoglycans, rheumatoid factor and citrullinated proteins) can play a role in the chronicity of the process.

### Pathogenesis of the disease

The synovium is a membrane source mesenchimiale formed by synoviocytes type 1 (macrophage) and type 2 (fibrinoid) and in disease undergoes hyperplasia and hypertrophy. It grows in thickness (usually consists of 2 or at most 3 layers of cells in the disease become 7 or more) and is formed so that the pannus begins peripherally to erode the bone not covered by cartilage (bare bone, "bare bone "). At the same time polimorfinucleati moving in the synovial fluid and lymphocytes T, B and plasma cells at the synovium-like tissue form a lymph node. The cells of the synovial lining take on a neoplastic-like and are not affected by contact inhibition.

The typical symptoms are pain, swelling warm but not red, functional impotence of the joints, typically the proximal interphalangeal (IFP) and / or metacarpophalangeal (MCP).

These symptoms are related to the biological circadian rhythm. In fact, in healthy subjects the level of pro-inflammatory cytokines (such as TNF-alpha and interleukin IL-6) increases during the night to reach the peak in the early morning. In addition, cytokines activate the hypothalamic-pituitary-adrenal axis, inducing metabolic changes typical of chronic inflammatory conditions of rheumatoid arthritis. Therefore, phenomena such as stiffness, pain and swelling Hot vary throughout the day, occurring mostly during the early hours of the morning.

The inflammation can also affect the tendons and there are different clinical manifestations, "finger swan neck" with hyperextension of the proximal interphalangeal joint (IFP) and flexion of the distal interphalangeal joint (IFD); "finger on the button in the loop" or en boutonniere with flexion and hyperextension of the IFP IFD; or"hammer toe" with an extensor tendon injury that can result in fixed flexion of the distal phalanx.
Another feature is the Baker's cyst at the level of the popliteal fossa which can break, creating bruising.

The disease is systemic so it can involve other organs and systems. Typical are rheumatoid nodules, superficial or deep, which may also occur in the lung. There may therefore be pulmonary fibrosis, pleurisy and pleuropericarditis. The cardiac addition there may be an acceleration of atherosclerosis of the coronary arteries. At eye level you can have xerophthalmia, uveitis and scleritis. Different are the iatrogenic pathologies while complications of RA are amyloidosis and osteoporosis. In addition, there is an increase in ESR, CRP, fever and general malaise.

There are four clinical variants:
Caplan's syndrome, characterized by a lung involvement, with a pulmonary nodular, linked to exposure to asbestos, silica and charcoal. The rheumatoid nodules in the presence of the above agents increase in size and may coalesce and form of the excavations.
Felty's syndrome, which adds to the typical manifestations of rheumatoid arthritis the presence of splenomegaly and leukopenia. Still's disease, the association with the presence of a macular rash high fever, but with a generally fleeting and non-erosive polyarthritis. Malignant Rheumatoid Arthritis, particularly severe form with vasculitic involvement widespread and important bony erosions.

The state of the disease can be identified by analyzing the type of injury to the patient:
Stage 1: There is an infiltration of CD4 + lymphocytes and macrophages, can be seen macroscopically symmetric swelling, no redness, there are systemic symptoms and rheumatoid nodules. Notes increase in the circulation of inflammatory markers and rheumatoid factor
Stage 2: You notice inflammation and synovial and endothelial proliferation (angiogenesis and pannus formation), the anomaly is seen on ultrasound as hypoechoic areas; on the contrary hyperplastic areas are hyperechoic. There are also erosion of the bone, resorption of cartilages and tendons rupture. The bone changes are seen in X-rays and even better in ultrasound. From this stage, the synovial hyperplasia is irreversible.
Stage 3: There are bone deformities, dislocations and fibrosis evident. The course is varied and generally characterized by periods of exacerbation and remission. There are milder forms that respond well to current treatments and serious forms which run without remission, leading to ankylosis and paintings serious functional impairment; in many cases the disease is not serious because it would endanger the life, but because, by preventing the proper use of the limbs, especially the hands and feet, involves a high degree debilitating, currently evaluated clinically with the EDSS (Expanded Disabilty Status Scale page), where those affected, may find it difficult not only in practice but also in the care of his person.

In general, early diagnosis is important because in the very first months of the onset of disease is observed substantial damage and irreversible. Also in the first two years of disease after diagnosis, the damage are particularly severe. The impairment of the joints leads to a limitation of mobility that can result in disability and subsequent premature death.

As clinical criteria requires the presence of at least four of these criteria in order to formulate a probable diagnosis of rheumatoid arthritis:
- morning stiffness lasting at least 1 hour;
- arthritis at the level of 3 or more joints;
- arthritis of the joints of the hand;
- symmetrical arthritis;
- cutaneous rheumatoid nodules;
- A positive test for rheumatoid factor;
- Radiological changes.

The most useful test for the diagnosis of rheumatoid arthritis are anti-citrulline antibodies (CCP), rheumatoid factor, ESR, CRP.

Other manifestations are not pathognomonic Rheumatoid Arthritis:
- Carpal tunnel syndrome (because the involvement of MCF damages the median nerve)
- vasculitis
- Sjögren's syndrome
- amyloidosis
- alterations in the pleura and pericardium

At the date 2014 there is no known pharmacological treatment that involves a final shutdown of inflammation nor a restoration of joint function.

At a mechanical level, you can resort to physical therapy to alleviate the effects in advanced stages by a surgery to remove the pannus too exuberant (synoviectomy), practice arthroplasty and arthrodesis. In addition, the eventual recovery of joint function can be assessed only in the surgical phase of the submissive disease. The interventions are invasive and take place in the reconstruction of bone and cartilage with the introduction of prosthesis. In general, do not solve the problem to the joint and can become potential effect of risk in the event of reactivation of the disease. Furthermore, they should be subject to periodic inspections and is not guaranteed in general, the success of the intervention.

The drugs used in the treatment of RA are varied, the properties and their side effects in the short and long term are now known as the 90% of them are used for many years now. The therapeutic approach has changed considerably over time as they tend to attack the disease since its debut, with drugs that were previously used as a last resort.

The therapy is based on two classes of drugs, "symptomatic" and "bottom".

Among the drugs "symptomatic" are: aspirin, paracetamol, fans (NSAIDs) and corticosteroids (anti-inflammatory drugs); drugs that contain the pain and inflammation but does not change the evolution of the disease.

They belong to the class of "background" medications, those substances that modify the clinical course of the disease and slow down in time the evolution of the anatomical damage of the joints; among these are gold salts, antimalarials, d-penicillamine, sulfalazina, immunosuppressants (methotrexate, cyclosporin A, Arava) in addition to the latest research have lengthened the list of drugs background with biologies or Anti tumor necrosis factor (TNFa) currently used on people with RA after traditional treatments have virtually failed.

Below we have provided an overview of current drug therapies most used and their respective side effects:
A) Drugs for mild forms of initial and Rheumatoid
   Arthritis: Anti-inflammatory and antirheumatic drugs
B) the most aggressive and advanced stages of the disease: Methotrexate: Patients treated with methotrexate should be monitored constantly because of the high toxicity of the drug at the level of various organs including bone marrow, liver, kidneys, lungs. In case of occurrence of adverse effects it is necessary to reduce the dosage and co-administered with folinic acid or discontinue therapy.

The Leflunomide is the basic substance that makes up the Arava, inhibits pyrimidine synthesis; rheumatologist prescribed by means of a treatment plan, is the alternative to Methotrexate as it is used in the forms of rheumatoid arthritis resistant.

Biologics (monoclonal antibodies), which act in a more selective and specific, and these are:
Adalimumab, Rituximab, Infliximab and Etanercept most used fusion protein anti-TNF alpha, documented clinical efficacy, trade name Enbrel. Currently represents a recovered easily used in combination with methotrexate, which however presents the same side effects. In some cases it has developed the onset of Hodgkin's disease although the cause-effect correlation is yet to be ascertained.

Is observed the occurrence of tumors and malignancies in patients treated with etanercept and immunosuppressants (methotrexate and corticosteroids).

Other therapeutic approaches:
Recent studies have found that some people with rheumatoid arthritis had relief from cannabis use, there is an experimental drug called Sativex (a cannabis extract containing tetrahydrocannabinol and cannabidiol).

### DISCLOSURE OF INVENTION

Building on a previous patent application (PCT / IT2014 / 000207), aiming at the recognition of a pharmacological treatment combined with atorvastatin, minocin and fluconazole, aimed at improving the quality of life of patients suffering from multiple sclerosis, applicants have been able to assess the effectiveness of the combined use of atorvastatin and minocycline which would produce a significant change in the conditions of lipid accumulation in the human being. Moreover, it has been seen that the association with acycloguanosine and the supplement of vitamin D3 made accumulations of synovial pannus decidedly softer, with significant reduction of the same and restor the proper articulation of the limbs affected by rheumatoid arthritis.

Besides the use of atorvastatin, the lipid-lowering effects of which are known, to be the same already commonly used to reduce the accumulation of cholesterol, the supplement of minocycline appears to be necessary since the same has an effective level of endo-cells catalyst, a role that is still more enhanced by the use of acycloguanosine, suitable to permanently remove the residues present in intracellular level of an alleged mononucleosis (diagnosed or not) and related strains probably remained inert in the body and grown up as a result of hormonal changes or stressors (in women it manifests itself either with menopause or during the period of greatest fertility, which would also explain the rare cases of complete remission after pregnancy. In men, consequently, could be involved in a moment of testosterone deficiency with a prevalence of 17beta estradiol).

The use of an implementation of vitamin D3 finds justification in the fact that since this is in fact a pro-hormone, is able to fix calcium in the bones, thus facilitating the recovery of damaged tissue by arthritis same. The thickening of the synovial pannus means that this will act as a sponge, preventing the passage of calcium, which is fixed in locations surrounding the joint, making the same dysfunctional, therefore the use of vitamin D3 in combination with other substances enables to ensure that the calcium is absorbed by the right target and not dispersed or fixed at the level of cartilage.

In the active phase of the disease the use of the drugs listed above could effectively reduce inflammation determined by the disease itself, thus avoiding the use of other substances such as painkillers or NSAIDs in general.

A good example of the above has been provided by a person, the wife of the second inventor of the present that suffering from rheumatoid arthritis for over twenty years, who, under medical supervision and following signing of a suitable confidentiality agreement, he wanted to personally experience the combination referred to below. The findings were nothing short of amazing, including the disappearance of pain, reduction in the synovial lining and the gradual absorption of rheumatoid nodules.

The novelty of what is claimed resides in having identified over to the actual operation of the two molecules (Atorvastatin and minocycline) mentioned above in association with each other (which when combined produce a synergistic effect and much higher than that of the individual), also the interaction of acycloguanosine and vitamin D3, which combined with the other fail to bring the situation of the patient in a phase of functional recovery with disease remission.

The subject referred to above, has decided to test the combination referred to below because of its low toxicity compared to drugs previously used (methotrexate and Enbrel) which did not provide a real effect in the sense of the remission of the disease. The patient showed a significant functional recovery as early as the first week of dosing with total absence of pain despite the lack of intake of other

### substances.

To date, the patient involved has a significant improvement in quality of life, having recovered much of her autonomy, which must still be considered in relation to the starting point of taking drug based on the EDSS. In any case, in the industrial production must be indicated the potential side effects, warnings and precautions for use.

### BEST MODE TO CARRYING THE INVENTION

The dosage of the treatment applied and proposed is as follows:
intake for 45 days in a combination of 100 mg of minocycline, 20 mg of atorvastatin, 200 mg of acycloguanosine, 400 IU of vitamin D, included in a capsule to be taken, considering the half-life of the substance, every 12 hours.

The best way to prepare it, even for a hypothetical industrial applicability, seems to be a gastro-resistant capsule inclusive of all four of the active ingredients.

None of the above drugs interacted adversely with any drug already used by patients and none of them had a relapse during treatment. It is noted, however, preferable to suspend the current treatments with other drugs, especially methotrexate (chemotherapeutic or immunosuppressive agents and in general) and immuno-modulating.

To date, given the proven effectiveness of the individual components, the low toxicity of the same and the long stay in the market, as well as the increased efficiency observed from the combination of the above used according to the new pattern of use over-exposed, it can be said that the new composition referred to above comprising the combination of minocycline with acycloguanosine, atorvastatin and vitamin D3, is effective in reducing the incidence of the disease on quality of life of the patient.

## Claims

1. A combination of atorvastatin, minocycline, acycloguanosine and vitamin D3 for use in the preparation of a single composition for the treatment of autoimmune type of rheumatoid arthritis, in form of a pill, capsule or tablet.;

2. A combination comprising 100 mg minocycline, 20 mg atorvastatin, 200 mg acycloguanosine, 400IU vitamin D3 for use in improving the quality of life in patients with rheumatoid arthritis, wherein said composition is for oral administration every 12 hours in form of a pill, capsule or tablet either as a single composition or by summation of the four single substances;

## Patentansprüche

1. Eine Kombination von Atorvastatin, Minocyclin, Acycloguanosin und Vitamin D2 für das Präparat einer einzigen Zusammensetzung für die Behandlung von autoimmuner rheumatoider Arthritis, in Form von Tabletten, Kapseln oder Kompressen.

2. Eine Kombination von 100 mg Minocyclin, 20 mg Atorvastatin, 200 mg Acycloguanosin, 400IU Vitamin D3 für die Verbesserung der Lebensqualität von Patienten mit rheumatoider Arthritis, wobei die besagte Zusammensetzung alle 12 Stunden mündlich in Form einer Tablette, Kapsel oder Kompresse verabreicht wird, entweder als einzige Zusammensetzung oder als Addierung der vier einzelnen Substanzen.

## Revendications

1. Une combinaison d'atorvastatine, minocycline, acycloguanosine et vitamine D3 à utiliser dans la préparation d'un seul composé pour le traitement de la polyarthrite rhumatoïde du type auto-immune, sous forme de comprimé, capsule ou cachet ;

2. Une combinaison comprenant 100 mg de minocycline, 20 mg d'atorvastatine, 200 mg d'acycloguanosine, 400 IU de vitamine D₃ à utiliser pour améliorer la qualité de vie des malades de polyarthrite rhumatoïde, auxquels le dit composé est administré par voie orale toutes les 12 heures sous forme de comprimé, capsule ou cachet, en tant que composé unique ou par somme de chacune des quatre substances.
